# FASCICULE DE BREVET EUROPEEN

(11) **EP 0 661 095 B1**
(45) Date de publication et mention de la délivrance du brevet: **23.06.1999**
(21) Numéro de dépôt: 94402929.7
(22) Date de dépôt: 19.12.1994
(51) Int. Cl.: B01J 23/40, B01J 23/74, C10G 45/60

(54) **Catalyseur pour la réduction de la teneur en benzène dans les essences**
Katalysator zur Erniedrigung des Benzolgehaltes von Benzinen
Catalyst for lowering the benzene content in gasolines

(30) Priorité: 29.12.1993 FR 9315953
(43) Date de publication de la demande: 05.07.1995
(73) Titulaire: INSTITUT FRANCAIS DU PETROLE, 92502 Rueil-Malmaison (FR)
(72) Inventeur: Travers, Christine, F-92500 Rueil Malmaison (FR); Courty, Philippe, F-78800 Houilles (FR); Sarrazin, Patrick, F-92500 Rueil Malmaison (FR)

(56) Documents cités:
- EP-A- 0 552 069
- WO-A-92/20759
- FR-A- 1 220 868
- US-A- 3 025 248
- US-A- 3 173 856

## Description

L'invention concerne d'une part, un catalyseur comprenant de 4 à 15 % d'au moins un halogène et notamment du chlore et au moins un métal du groupe VIII déposé sur un support constitué d'un mélange d'alumine éta et d'alumine gamma dans des proportions bien déterminées et d'autre part, un procédé, en présence dudit catalyseur, de réduction de la teneur en benzène dans les fractions essences dans lequel on effectue l'isomérisation d'une charge telle que du réformat léger et/ou une coupe C₅-C₆.

Les problèmes liés à l'environnement vont conduire conjointement à la réduction de la teneur en plomb et à la réduction de la teneur en benzène dans le pool essence, de préférence sans diminution d'indice d'octane. Le reformage catalytique utilisé dans des conditions de forte sévérité, et l'isomérisation des normales paraffines C₅-C₆ de faible indice d'octane sont les procédés les plus couramment utilisés pour obtenir des indices d'octane élevés sans adjonction de plomb. Cependant, le procédé de réformage catalytique produit des quantités importantes de benzène de haut indice d'octane. C'est pourquoi il est nécessaire de développer de nouveaux procédés permettant de réduire la teneur en benzène des carburants tout en satisfaisant aux spécifications sur l'indice d'octane.

La combinaison des procédés de reformage catalytique et d'isomérisation, consistant à séparer la fraction C₅-C₆ du réformat, à l'isomériser et à l'introduire directement dans le pool essence pour améliorer l'indice d'octane est bien connue : elle est décrite par exemple dans les brevets US-A-4 57 832, US-A-4 181 599, US-A-3 761 392. Le traitement par isomérisation de la coupe C₅-C₆ issue de la distillation directe du pétrole brut est également bien connu. Il conduit à une amélioration considérable de l'indice d'octane de cette coupe. La réduction de la teneur en benzène du réformat peut également être effectuée de différentes façons, telles que par exemple la modification du point de coupe du naphta entre le reformage et l'isomérisation, ou la séparation du réformat en deux fractions une fraction lourde (un réformat lourd) et une fraction légère (réformat léger) tout le benzène étant concentré dans ladite fraction légère. Cette fraction légère est ensuite envoyée dans une unité d'hydrogénation qui permet de transformer le benzène en naphtène, qui est ensuite décyclisé dans une unité d'isomérisation travaillant dans des conditions sévères. Les normales paraffines ainsi formées sont isomérisées par un procédé classique d'isomérisation (US-A-5 003 118). Par ailleurs, la demande de brevet EP-A-552070 concerne un procédé de réduction de la teneur en benzène dans les fractions essences dans lequel on effectue une hydrogénation de la charge caractérisée par une composition pondérale comprise dans les intervalles suivants : entre 40 et 80 % de paraffines, entre 0,5 et 7 % d'hydrocarbures cycliques et entre 6 et 45 % d'aromatiques et par une température maximale de distillation comprise entre 70 et 90 °C, puis une isomérisation de l'effluent issu de l'hydrogénation, avec mélange à ladite charge et/ou audit effluent d'une coupe C₅-C₆.

La demande de brevet EP-A-552069, elle, concerne un procédé d'isomérisation d'une charge telle que du réformat léger et/ou d'une coupe C₅-C₆, en présence d'un catalyseur d'isomérisation qui comprend de préférence au moins un métal du groupe VIII et une mordénite de rapport Si/Al compris entre 5 et 50.

Le brevet US-A-3,025,248 décrit le procédé de fabrication d'un catalyseur dont le support à base d'alumines alpha et bêta est traité de façon à convertir ces alumines en alumines êta et gamma. Le catalyseur contient également une petite quantité de platine ou d'un autre métal tel que le palladium, le nickel, le fer ou le cobalt, et éventuellement du chlore à l'état de traces.

La présente invention concerne un catalyseur, utilisable notamment dans un procédé de réduction de la teneur en benzène dans les fractions essences.

Un tel catalyseur est à base d'un support constitué d'alumine éta et d'alumine gamma, la teneur en alumine éta étant comprise entre 85 et 95 % poids, de préférence entre 88 et 92 % poids et de manière encore plus préférée entre 89 et 91 % poids, le complément à 100 % poids du support étant sensiblement constitué d'alumine gamma, ledit catalyseur comprenant aussi de 4 à 15 %, de préférence 6 à 15 %, de préférence encore 6 à 11 %, d'au moins un halogène, de préférence le chlore, et au moins un métal du groupe VIII.

L'alumine éta utilisée dans la présente invention a une surface spécifique généralement comprise entre 400 et 600 m²/g, de manière préférée entre 420 et 550 m²/g, et un volume poreux total généralement compris entre 0,3 et 0,5 cm³/g, de manière préférée entre 0,35 et 0,45 cm³/g.

L'alumine gamma utilisée dans la présente invention possède une surface spécifique généralement comprise entre 150 et 300 m²/g, de préférence entre 180 et 250 m²/g, et un volume poreux total généralement compris entre 0,4 et 0,8 cm³/g, de manière préférée entre 0,45 et 0,7 cm³/g.

Les deux types d'alumine sont mélangés et mis en forme dans les proportions définies ci-avant, par toute technique connue de l'homme du métier, par exemple par extrusion au travers d'une filière, par pastillage ou dragéification.

Le support ainsi obtenu a une surface spécifique généralement comprise entre 300 et 550 m²/g, de préférence entre 350 et 500 m²/g et un volume poreux généralement compris entre 0,3 et 0,6 cm³/g, de préférence entre 0,35 et 0,5 cm³/g.

Au moins un métal hydrogénant du groupe VIII, de préférence choisi dans le groupe formé par le platine, le palladium, et le nickel, est ensuite déposé sur ce support par toute technique connue de l'homme du métier, par exemple par échange anionique sous forme d'acide hexachloroplatinique dans le cas du platine ou sous forme de chlorure dans le cas du palladium.

Dans le cas du platine ou du palladium, la teneur en poids est comprise entre 0,05 et 1 % et de manière préférée entre 0,1 et 0,6 %. Dans le cas du nickel la teneur pondérale est comprise entre 0,1 et 10 % et de manière préférée entre 0,2 et 5 %.

Le catalyseur ainsi préparé est réduit sous hydrogène, puis soumis à un traitement d'halogénation par tout composé halogéné connu de l'homme du métier. Dans le cas du chlore, un tel composé halogéné peut être le tétrachlorure de carbone ou le perchloréthylène. La teneur en halogène, de préférence le chlore, du catalyseur final est comprise entre 4 et 15 %, de préférence entre 6 et 15 %, de préférence encore entre 6 et 11 %.

Ce traitement d'halogénation peut être effectué directement dans l'unité avant injection de la charge ou hors site. Il est aussi possible de procéder au traitement d'halogénation préalablement au traitement de réduction sous hydrogène.

La présente invention concerne également l'utilisation de ce catalyseur dans un procédé notamment de réduction de la teneur en benzène dans les fractions essences dans lequel on effectue l'isomérisation d'une charge telle que la fraction légère du réformat et/ou une coupe C₅-C₆, généralement issue de la distillation directe. On obtient ainsi de façon surprenante un effluent pratiquement totalement exempt de benzène (c'est-à-dire comprenant moins de 0,1 % poids de benzène) et présentant un indice d'octane recherche supérieur ou égal à l'indice d'octane recherche du réformat léger, ce qui permet de l'incorporer directement aux fractions essences après stabilisation.

La fraction légère du réformat est obtenue par distillation dudit réformat. Elle est définie par une température maximale de distillation comprise entre 70 et 90°C, de manière préférée entre 77 et 83 °C, et une composition pondérale par familles d'hydrocarbures comprise dans les intervalles suivants : entre 40,0 et 80,0% de paraffines, entre 0,5 et 7,0 % d'hydrocarbures cycliques (tel que le méthylcyclopentane, le cyclopentane ou le cyclohexane), et entre 6,0 et 45,0 % d'aromatiques. La température de distillation est généralement comprise entre la température ambiante et la température maximale de distillation (ou température de tête).

La famille des hydrocarbures aromatiques est généralement essentiellement constituée de benzène. Par ailleurs entre 1,0 et 3,0 % d'hydrocarbures oléfiniques peuvent être présents dans ladite fraction légère. Généralement, ladite fraction légère possède de plus les caractéristiques suivantes : le poids moléculaire moyen de ladite fraction est compris entre 70 et 90 g/mol, la masse volumique mesurée à 15 °C est comprise entre 0,670 et 0,780 g/cm³ et la valeur de l'indice d'octane recherche est compris entre 75 et 90.

Tout autre charge hydrocarbonée provenant d'un autre procédé ou ensemble de procédés et définie comme ci-dessus c'est-à-dire par une composition pondérale comprise dans les intervalles suivants : entre 40 et 80 % de paraffines, entre 0,5 et 7 % d'hydrocarbures cycliques et entre 6 et 45 % d'aromatiques, et par une température maximale de distillation comprise entre 70 et 90 °C, de préférence entre 77 et 83 °C, peut également être utilisée.

La composition pondérale de ladite coupe C₅-C₆ annoncée ci-dessus et généralement issue de la distillation directe dépend de la nature du brut à traiter.

Néanmoins, ladite coupe est généralement définie par une teneur en paraffines généralement supérieure à 90 % poids, une teneur en hydrocarbures cycliques généralement inférieure à 10 % poids et une teneur en benzène généralement inférieure à 1,5 % poids. Son indice d'octane recherche est généralement compris entre 60 et 75.

D'autre part, ladite coupe peut contenir de très faibles teneurs de composés comportant 4 atomes de carbone par molécule (moins de 0,5 % poids).

Selon la présente invention les deux charges décrites ci-dessus sont mélangées puis envoyées ensemble à l'unité d'isomérisation, la teneur en coupe C₅-C₆ dans le mélange variant de 10 à 90 % et de manière préférée de 15 à 55 %.

On ajoute généralement au mélange obtenu précédemment un composé chloré tel que le tétrachlorure de carbone ou le perchloréthylène, avant l'entrée en zone d'isomérisation, de façon que la teneur en chlore dans la charge soit comprise entre 50 et 5000 ppm et de préférence entre 100 et 1000 ppm.

La zone d'isomérisation est mise en oeuvre dans les conditions usuelles de l'isomérisation. Elle est comprise dans au moins un réacteur.

La température est comprise entre 100 et 300°C et de préférence entre 120 et 250° C, et la pression partielle d'hydrogène est comprise entre la pression atmosphérique et 70 bar et de préférence entre 5 et 50 bar. La vitesse spatiale est comprise entre 0,2 et 10 litres et de préférence entre 0,5 et 5 litres d'hydrocarbures liquides par litre de catalyseur et par heure. Le rapport molaire hydrogène/charge à l'entrée du réacteur est tel que le rapport molaire hydrogène/charge dans l'effluent est supérieur à 0,06, de préférence compris entre 0,06 et 10.

L'effluent stabilisé ainsi obtenu présente alors un indice d'octane suffisamment élevé pour être incorporé au pool essence après stabilisation et est pratiquement totalement exempt de benzène (teneur dans l'effluent inférieure à 0,1 % poids).

Les exemples qui suivent précisent l'invention sans en limiter la portée.

### EXEMPLE 1 (selon l'invention)

Un réformat léger obtenu après distillation à 80 °C, contenant 21,5 % de benzène et présentant un indice d'octane de 80,3 est mélangé à raison de 50 % poids avec une coupe C₅-C₆ de distillation directe contenant 0,7 % de benzène et présentant un indice d'octane de 65. Les compositions de ces deux produits figurent dans le tableau 1. Le réformat léger comprend donc en poids 21,5 % d'aromatiques, 4 % d'hydrocarbures cycliques et 74,5 % de paraffines. La coupe C₅-C₆ comprend en poids 0,7 % de benzène, 7,25 % d'hydrocarbures cycliques et 92,05 % de paraffines. Le mélange de ces deux charges, dont la composition figure également dans le tableau 1, est envoyé dans une unité d'isomérisation à une température de 170 °C, une pression de 30 bar avec une vitesse spatiale égale à 2 litres d'hydrocarbures liquides par litre de catalyseur et par heure et un rapport molaire hydrogène/hydrocarbures de la charge tel que ce même rapport est égal à 0,07 dans l'effluent.

Le catalyseur utilisé est composé de 0,3 % poids de Pt déposé sur un support constitué de 90 % poids d'alumine éta et de 10 % poids d'alumine gamma. Le catalyseur ainsi défini est ensuite chloré à raison de 9 % poids de Cl. L'effluent sorti de l'unité d'isomérisation a la composition donnée au tableau 1. Il ne contient pratiquement plus de benzène et présente un indice d'octane de 81,5. Il est donc directement incorporable dans les fractions essence après stabilisation.

**Tableau 1**

| | **Réformat** | **Coupe C**_{**5**}**-C**_{**6**} **de distillation** | **Charge entrée isomérisation** | **Effluent sortie isomérisation** |
|---|---|---|---|---|
| Légers | 6,5 | 1,0 | 3,7 | 6,0 |
| iC₅ | 9,9 | 18,9 | 14,4 | 23,7 |
| nC₅ | 7,1 | 25,4 | 16,25 | 7,4 |
| 22DMC₄ | 3,0 | 0,4 | 1,7 | 13,2 |
| 23DMC₄ | 4,1 | 1,85 | 3,0 | 4,9 |
| 2MC₅ | 15,8 | 11,1 | 13,45 | 16,5 |
| 3MC₅ | 12,5 | 9,4 | 11,0 | 10,1 |
| nC₆ | 12,1 | 19,6 | 15,9 | 6,7 |
| C₇ | 3,5 | 4,4 | 3,9 | 3,0 |
| CC₅ | 0,4 | 1,4 | 0,9 | 0,5 |
| MCC₅ | 3,6 | 4,1 | 3,85 | 4,9 |
| CC₆ | 0 | 1,75 | 0,85 | 3,1 |
| Benzène | 21,5 | 0,7 | 11,1 | - |
| R.O.N. | 80,3 | 65 | 72,9 | 81,5 |

### EXEMPLE 2 (selon l'invention)

On prépare six catalyseurs référencés de A à F, composés de 0,3 % de Pt déposé sur un support comprenant un mélange d'alumine éta et d'alumine gamma, la teneur en alumine éta variant de 85 à 95 % dans ce support, comme indiqué tableau 2. Les catalyseurs ainsi définis sont chlorés à raison de 9 % poids de chlore. La charge d'entrée de l'isomérisation dont la composition figure tableau 1 est envoyée à une unité d'isomérisation fonctionnant dans les conditions décrites dans l'exemple 1. Les RON obtenus après isomérisation sont donnés tableau 2. On constate que le maximum de RON est obtenu pour une teneur en alumine éta dans le support comprise entre 89 et 91 %.

**Tableau 2**

| | **A** | **B** | **C** | **D** | **E** | **F** |
|---|---|---|---|---|---|---|
| Teneur en Al₂O₃ η dans le support (%) | 85 | 88 | 89 | 91 | 92 | 95 |
| RON après isomérisation | 81 | 81,2 | 81,5 | 81,5 | 81,2 | 81 |

### EXEMPLE 3 (selon l'invention)

Le présent exemple diffère de l'exemple 1 uniquement en ce que le catalyseur défini comme dans l'exemple 1, est chloré à raison de 7 % poids de chlore.

L'effluent sorti de l'unité d'isomérisation à la composition donnée tableau 3. Il ne contient plus de benzène et présente un indice d'octane de 80,3. Il est donc directement incorporable dans les fractions essence après stabilisation.

**Tableau 3**

| | **Réformat** | **Coupe C**_{**5**}**-C**_{**6**} **de distillation** | **Charge entrée isomérisation** | **Effluent sortie isomérisation** |
|---|---|---|---|---|
| Légers | 6,5 | 1,0 | 3,7 | 6,0 |
| iC₅ | 9,9 | 18,9 | 14,4 | 22,2 |
| nC₅ | 7,1 | 25,4 | 16,25 | 8,9 |
| 22DMC₄ | 3,0 | 0,4 | 1,7 | 12,7 |
| 23DMC₄ | 4,1 | 1,85 | 3,0 | 4,5 |
| 2MC₅ | 15,8 | 11,1 | 13,45 | 16,5 |
| 3MC₅ | 12,5 | 9,4 | 11,0 | 10,1 |
| nC₆ | 12,1 | 19,6 | 15,9 | 7,6 |
| C₇ | 3,5 | 4,4 | 3,9 | 3,0 |
| CC₅ | 0,4 | 1,4 | 0,9 | 0,5 |
| MCC₅ | 3,6 | 4,1 | 3,85 | 4,9 |
| CC₆ | 0 | 1,75 | 0,85 | 3,1 |
| Benzène | 21,5 | 0,7 | 11,1 | - |
| R.O.N. | 80,3 | 65 | 72,9 | 80,3 |

### EXEMPLE 4 (comparatif)

L'exemple 4 diffère de l'exemple 3, uniquement en ce que le catalyseur utilisé dans la zone d'isomérisation est composé de 0,3 % poids de Pt déposé sur un support constitué de 50 % poids d'alumine éta et de 50 % poids d'alumine gamma. Le catalyseur ainsi défini est ensuite soumis à un traitement de chloration. La teneur finale en chlore est de 7 % poids.

Le tableau 4, donne la composition de l'effluent sorti de l'unité d'isomérisation.

**Tableau 4**

| | **Réformat** | **Coupe C**_{**5**}**-C**_{**6**} **de distillation** | **Charge entrée isomérisation** | **Effluent sortie isomérisation** |
|---|---|---|---|---|
| Légers | 6,5 | 1,0 | 3,7 | 65,5 |
| iC₅ | 9,9 | 18,9 | 14,4 | 18,4 |
| nC₅ | 7,1 | 25,4 | 16,25 | 12,3 |
| 22DMC₄ | 3,0 | 0,4 | 1,7 | 10,3 |
| 23DMC₄ | 4,1 | 1,85 | 3,0 | 3,9 |
| 2MC₅ | 15,8 | 11,1 | 13,45 | 12,9 |
| 3MC₅ | 12,5 | 9,4 | 11,0 | 7,9 |
| nC₆ | 12,1 | 19,6 | 15,9 | 15,0 |
| C₇ | 3,5 | 4,4 | 3,9 | 3 |
| CC₅ | 0,4 | 1,4 | 0,9 | 0,5 |
| MCC₅ | 3,6 | 4,1 | 3,85 | 5,2 |
| CC₆ | 0 | 1,75 | 0,85 | 5,1 |
| Benzène | 21,5 | 0,7 | 11,1 | - |
| R.O.N. | 80,3 | 65 | 72,9 | 74,8 |

Le gain d'indice d'octane (RON) entre la charge de l'unité d'isomérisation et son effluent est très faible. Il y a perte d'indice d'octane (RON) par rapport au réformat léger.

Note : dans les tableaux ci-dessus :
- 22DMC₄: = 2,2-diméthylbutane
- 23DMC₄: = 2,3-diméthylbutane
- 2MC₅: = 2-méthylpentane
- 3MC₅: = 3-méthylpentane
- CC₅: = cyclopentane
- MCC₅: = méthyl cyclopentane
- CC₆: = cyclohexane

## Revendications

1. Catalyseur comprenant de 4 à 15% d'au moins un halogène et au moins un métal du groupe VIII sur un support qui est sensiblement constitué d'alumine éta et d'alumine gamma, la teneur en alumine éta étant comprise entre 85 et 95% poids par rapport au support, le complément à 100% poids, du support étant de l'alumine gamma.

2. Catalyseur selon la revendication 1 dans lequel ladite teneur en alumine éta du support du catalyseur est comprise entre 88 et 92% poids.

3. Catalyseur selon l'une des revendications 1 et 2 dans lequel le métal du groupe VIII est choisi dans le groupe formé par le platine, le palladium et le nickel.

4. Catalyseur selon l'une des revendications 1 à 3 dans lequel l'alumine éta a une surface spécifique comprise entre 400 et 600 m²/g et un volume poreux compris entre 0,3 et 0,5 cm³/g.

5. Catalyseur selon l'une des revendications 1 à 4 dans lequel l'alumine gamma a une surface spécifique comprise entre 150 et 300 m²/g et un volume poreux compris entre 0,4 et 0,8 cm³/g.

6. Catalyseur selon l'une des revendications 1 à 5 dans lequel la teneur en halogène est de 6 à 15%.

7. Catalyseur selon l'une des revendications 1 à 6 dans lequel l'halogène est le chlore.

8. Utilisation d'un catalyseur selon l'une des revendications 1 à 7 dans un procédé de réduction de la teneur en benzène dans les fractions essences.

9. Procédé de réduction de la teneur en benzène dans les fractions essences en présence d'un catalyseur selon l'une des revendications 1 à 7 dans lequel on effectue une isomérisation.

10. Procédé selon la revendication 9 dans lequel on procède à l'isomérisation d'un mélange (a) d'une charge définie par une composition pondérale comprise dans les intervalles suivants : entre 40 et 80% de paraffines, entre 0,5 et 7% d'hydrocarbures cycliques et entre 6 et 45% d'aromatiques, et par une température maximale de distillation comprise entre 70 et 90°C, et (b) d'une coupe C₅-C₆ définie par une teneur en paraffines supérieure à 90% poids, une teneur en hydrocarbures cycliques inférieure à 10% poids et une teneur en benzène inférieure à 1,5% poids.

11. Procédé selon l'une des revendications 9 ou 10 dans laquelle l'isomérisation se fait selon les conditions opératoires suivantes : la température est comprise entre 100 et 300°C, la pression partielle d'hydrogène est comprise entre la pression atmosphérique et 70 bar, la vitesse spatiale est comprise entre 0,2 et 10 litres de charge par litre de catalyseur et par heure, le rapport molaire hydrogène/charge à l'entrée du réacteur est tel que le rapport hydrogène/charge dans l'effluent est supérieur à 0,06 et on a ajouté à la charge un composé chloré de teneur comprise entre 50 et 5000 ppm.

12. Procédé selon l'une des revendications 9 à 11 dans lequel la coupe C₅-C₆ est une coupe de distillation directe.

13. Procédé selon l'une des revendications 9 à 12 dans lequel ledit mélange comprend 10 à 90% en poids de ladite coupe.

## Claims

1. A catalyst comprising 4 % to 15 % of at least one halogen and at least one group VIII metal on a support which essentially consists of eta alumina and gamma alumina, the eta alumina content being between 85 % and 95 % by weight with respect to the support, the complement to 100 % of the support being gamma alumina.

2. A catalyst according to claim 1, wherein said eta alumina content in the catalyst support is between 88 % and 92 % by weight.

3. A catalyst according to claim 1 or claim 2, wherein the group VIII metal is selected from the group constituted by platinum, palladium and nickel.

4. A catalyst according to any one of claims 1 to 3, wherein the eta alumina has a specific surface area of between 400 and 600 m²/g and a pore volume of between 0.3 and 0.5 cm³/g.

5. A catalyst according to any one of claims 1 to 4, wherein the gamma alumina has a specific surface area of between 150 and 300 m²/g and a pore volume of between 0.4 and 0.8 cm³/g.

6. A catalyst according to any one of claims 1 to 5, wherein the halogen content is 6 % to 15 %.

7. A catalyst according to any one of claims 1 to 6, wherein the halogen is chlorine.

8. Use of a catalyst according to any one of claims 1 to 7 in a process for reducing the benzene content of petrol fractions.

9. A process for reducing the benzene content of petrol fractions in the presence of a catalyst according to any one of claims 1 to 7 in which an isomerisation is performed.

10. A process according to claim 9 in which isomerisation is carried out on a mixture of a) a feed defined by the following composition by weight : 40 % to 80 % of paraffins, 0.5 % to 7 % of cyclic hydrocarbons and 6 % to 45 % of aromatics, and by a maximum distillation temperature of 70 °C to 90 °C, and b) a C₅-C₆ cut defined by a paraffin content of more than 90 % by weight, a cyclic hydrocarbon content of less than 10 % by weight and a benzene content of less than 1.5 % by weight.

11. A process according to claim 9 or 10 wherein isomerisation is carried out under the following operating conditions : a temperature of between 100 °C and 300 °C, a hydrogen partial pressure of between atmospheric pressure and 70 bar, a space velocity of between 0.2 and 10 litres of feed per litre of catalyst per hour, a hydrogen/feed molar ratio at the reactor inlet such that the hydrogen/feed molar ratio in the effluent is greater than 0.06, and that a chlorine compound is added to the feed in a concentration of between 50 and 5000 ppm.

12. A process according to anyone of claims 9 to 11, wherein the C₅-C₆ cut is a straight run cut.

13. A process according to any one of claims 9 to 12, wherein said mixture contains 10 % to 90 % of said cut.

## Patentansprüche

1. Katalysator, 4 bis 15% wenigstens eines Halogens und wenigstens ein Metall der Gruppe VIII auf einem Träger umfassend, der im wesentlichen gebildet wird durch Eta-Aluminiumoxid und Gamma-Aluminiumoxid, wobei der Gehalt an Eta-Aluminiumoxid zwischen 85 und 95 Gew.-% bezogen auf den Träger umfaßt und die Ergänzung zu 100 Gew.-% des Trägers Gamma-Aluminiumoxid ist.

2. Katalysator nach Anspruch 1, bei dem dieser Gehalt an Eta-Aluminiumoxid des Trägers des Katalysators zwischen 88 und 92 Gew.-% beträgt.

3. Katalysator nach einem der Ansprüche 1 und 2, bei dem das Metall der Gruppe VIII gewählt ist aus der durch Platin, Palladium und Nickel gebildeten Gruppe.

4. Katalysator nach einem der Ansprüche 1 bis 3, bei dem das Eta-Aluminiumoxid eine spezifische Oberfläche zwischen 400 und 600 m²/g und ein Porenvolumen zwischen 0,3 und 0,5 cm³/g hat.

5. Katalysator nach einem der Ansprüche 1 bis 4, bei dem das Gamma-Aluminiumoxid eine spezifische Oberfläche zwischen 150 und 300 m²/g und ein Porenvolumen zwischen 0,4 und 0,8 cm³/g hat.

6. Katalysator nach einem der Ansprüche 1 bis 5, bei dem der Gehalt an Halogen 6 bis 15% beträgt.

7. Katalysator nach einem der Ansprüche 1 bis 6, bei dem das Halogen Chlor ist.

8. Verwendung eines Katalysators nach einem der Ansprüche 1 bis 7 bei einem Verfahren zur Reduktion des Benzolgehaltes in den Benzinfraktionen.

9. Verfahren zur Reduzierung des Benzolgehaltes in den Benzinfraktionen in Anwesenheit eines Katalysators nach einem der Ansprüche 1 bis 7, bei dem man eine Isomerisierung durchführt.

10. Verfahren nach Anspruch 9, bei dem man eine Isomerisierung eines Gemisches (a) einer Charge vornimmt, die durch eine Gewichtszusammensetzung in den folgenden Intervallen definiert ist: zwischen 40 und 80% Paraffine, zwichen 0,5 und 7% zyklische Kohlenwasserstoffe und zwischen 6 und 45% Aromate und durch eine maximale Destillationstemperatur zwischen 70 und 90°C und (b) eines C₅-C₆ Schnittes, der durch einen Gehalt an Paraffinen von mehr als 90 Gew.-%, einen Gehalt an zyklischen Kohlenwasserstoffen von weniger als 10 Gew.-% und einen Gehalt an Benzol von weniger als 1,5 Gew.-% definiert ist.

11. Verfahren nach einem der Ansprüche 9 oder 10, bei dem die Isomerisierung unter den folgenden Arbeitsbedingungen abläuft: die Temperatur liegt zwischen 100 und 300°C, der Partialdruck des Wasserstoffes liegt zwischen dem atmosphärischen Druck und 70 bar, die Raumgeschwindigkeit liegt zwischen 0,2 und 10 Liter Charge pro Liter Katalysator und Stunde, wobei das Molverhältnis Wasserstoff/Charge am Eintritt des Reaktors derart ist, daß das Verhältnis Wasserstoff/Charge im Abstrom über 0,06 liegt und man der Charge eine chlorierte Verbindung mit einem Gehalt zwischen 50 und 5000 ppm zugesetzt hat.

12. Verfahren nach einem der Ansprüche 9 bis 11, bei dem der C₅-C₆ Schnitt ein Schnitt der direkten Destillation ist.

13. Verfahren nach einem der Ansprüche 9 bis 12, bei dem dieses Gemisch 10 bis 90 Gew.-% dieses Schnitts umfaßt.
